Europäisches Patentamt

European Patent Office

Office européen des brevets

(11) Numéro de publication: **0 350 355 B1**

(12) **FASCICULE DE BREVET EUROPEEN**

(45) Date de publication de fascicule du brevet: **23.03.94**

(21) Numéro de dépôt: **89401729.2**

(22) Date de dépôt: **19.06.89**

(51) Int. Cl.5: **C12P 7/52**, //(C12P7/52, C12R1:145)

Le dossier contient des informations techniques présentées postérieurement au dépôt de la demande et ne figurant pas dans le présent fascicule.

(54) **Procédé de production d'acide butyrique normal par fermentation à partir d'un substrat sucré en présence d'au moins une souche de l'espèce clostridium.**

(30) Priorité: **06.07.88 FR 8809273**

(43) Date de publication de la demande:
**10.01.90 Bulletin 90/02**

(45) Mention de la délivrance du brevet:
**23.03.94 Bulletin 94/12**

(84) Etats contractants désignés:
**AT BE CH DE ES GB IT LI NL**

(56) Documents cités:
**GB-A- 337 153**
**US-A- 1 875 688**
**US-A- 1 875 689**

(73) Titulaire: **INSTITUT FRANCAIS DU PETROLE**
**4, Avenue de Bois Préau**
**F-92502 Rueil-Malmaison(FR)**

(72) Inventeur: **Fayolle, Françoise**
**9 rue de Rouet-Pepion**
**F-92240 Malakoff(FR)**
Inventeur: **Michel, Dominique**
**32 A, avenue Jean Jaurès**
**F-92290 Chatenay Malabry(FR)**
Inventeur: **Marchal, Rémy**
**70, rue des Cormiers**
**F-78400 Chatou(FR)**
Inventeur: **Ballerini, Daniel**
**103, rue du Pontel**
**F-78100 St Germain en Laye(FR)**

EP 0 350 355 B1

Rank Xerox (UK) Business Services
(3.10/3.09/3.3.3)

## Description

L'invention concerne un procédé de production d'un mélange d'acides organiques contenant une majeure partie d'acide butyrique normal par fermentation à partir d'un substrat sucré en présence d'au moins une souche de l'espèce Clostridium.

Elle s'applique particulièrement à l'industrie des arômes et parfums.

L'arrière-plan technologique est illustré notamment par les brevets GB-A-337.153, US-A-1.875.689 et US-A-1.875.688.

Il est connu depuis très longtemps (FR 759 495, US 2549765) selon l'art antérieur de préparer de l'acide butyrique normal en batch (discontinu) par fermentation en présence d'un mélange de souches bactériennes contenant entre autres, Clostridium butyricum, à partir d'un substrat sucré. Le principal inconvénient de cette préparation est relatif au mélange d'acides obtenus. En effet, avec un substrat sucré contenant des vinasses, des mélasses et des écumes de sucrerie, on obtient selon le document français après 5 à 7 jours de fermentation, un mélange d'acides de composition suivante :

| - acide acétique | 15 à 10 % |
| - acide propionique | 35 à 30 % |
| - acide butyrique | 45 à 54 % |
| - acides supérieurs | 5 à 6 % |

Il s'ensuit donc une baisse en rendement en acide butyrique par rapport au substrat carboné fourni et des difficultés qui ont trait au stade de la purification et de la séparation. De plus, la durée de la fermentation pouvait varier entre 5 et 10 jours.

On a découvert un procédé de préparation d'acide butyrique normal améliorant notamment la sélectivité de la réaction et donc la production en acide butyrique recherché.

De manière plus détaillée, l'invention concerne un procédé de production, dans une zone réactionnelle, d'un mélange d'acides organiques ou des sels correspondants contenant une majeure partie d'acide n-butyrique ou de son sel correspondant, par fermentation dans des conditions de fermentation, d'un milieu réactionnel initial contenant un substrat sucré, en présence d'au moins une souche appartenant à l'espèce Clostridium, caractérisé en ce que :

a) on alimente ladite souche en ledit substrat à une concentration initiale comprise entre 1 et 80 g de substrat par litre de milieu réactionnel initial,

b) on laisse diminuer la concentration en substrat d'au moins 20 % et jusqu'à une valeur telle qu'elle soit comprise entre 0 et 30 g de substrat par litre de milieu réactionnel initial,

c) on alimente ladite souche en ledit substrat à un débit compris entre 0,01 et 25 g par litre de milieu réactionnel initial et par heure et pendant une durée d'alimentation tels que la concentration résiduelle en ledit substrat pendant la durée d'alimentation est au plus égale à 80 g/l de milieu réactionnel initial, de préférence comprise entre 0 et 40 g/l dudit milieu, et

d) on récupère ledit mélange d'acides ou de sels ainsi produit.

Par mélange d'acides organiques contenant une majeure partie d'acide n-butyrique, on entend un mélange comprenant généralement au moins 80 % à 100 % d'acide n-butyrique et 20 à 0 % d'acide acétique. On a obtenu avantageusement un mélange comprenant 90 à 100 % et de préférence de 95 % à 100 % d'acide n-butyrique, ce qui permet d'obtenir par la suite une purification plus aisée d'acide n-butyrique.

On peut utiliser avantageusement une souche de Clostridium choisie parmi Clostridium tyrobutyricum, Clostridium butyricum, Clostridium pasteurianum, Clostridium acetobutylicum et Clostridium beijerinckii. On a observé d'excellents résultats en opérant de préférence en présence de souche Clostridium tyrobutyricum en particulier des souches Clostridium tyrobutyricum I-776 et I-775, la sélectivité en acide n-butyrique pouvant atteindre 100 %. On a observé de plus que Clostridium tyrobutyricum CNRZ 596 pouvait utiliser comme hydrates de carbone non seulement le fructose et le glucose qu'utilise la souche I-776 mais aussi le mannose et le xylose.

Le milieu réactionnel comprend en général un substrat sucré comprenant des hydrates de carbone sous forme monosaccharides tels que le glucose et le fructose, provenant de l'hydrolyse acide ou enzymatique, dans des conditions connues de l'Homme de métier, des disaccharides et/ou polysaccharides contenus dans les matières premières telles que les jus sucrés obtenus par pressage ou diffusion de betteraves, de la canne à sucre et/ou du topinambour, les sirops, les égouts et les mélasses de sucrerie, les farines de céréales (blé, orge), les farines de maïs et la fécule de pommes de terre.

Les concentrations initiales en monosaccharides sont avantageusement comprises entre 1 et 30 g/l de milieu réactionnel initial et de manière préférée comprise entre 2 et 10 g/l.

Selon une caractéristique du procédé selon l'invention, on laisse diminuer la concentration en substrat sucré du fait de l'activité métabolique de la souche et de son développement cellulaire à une valeur avantageusement comprise entre 0 et 15 g/l de milieu réactionnel initial et de préférence comprise entre 0 et 6 g/l. Lorsque cette concentration résiduelle est atteinte, on fait démarrer l'alimentation en substrat sucré. De bons résultats de productivité ont été atteints lorsque le débit d'alimentation est avantageusement compris entre 2 et 20 g par litre de milieu réactionnel initial et par heure. Avec un débit d'alimentation préféré de 5 à 15 g/l/heure, on a obtenu une productivité pouvant être améliorée d'un facteur compris entre 2 et 3 par rapport à l'art antérieur et on a observé de manière surprenante que la sélectivité dans ces conditions avoisinait 100 %.

Le débit d'alimentation peut soit être maintenu constant durant toute la durée de l'alimentation soit varier de manière continue et/ou discontinue dans les limites décrites ci-dessus. De préférence, on injectera le substrat avec un débit asservi à la vitesse de la réaction, donc à la vitesse de dégagement gazeux et donc à l'activité fermentaire de la souche durant toute la durée de l'étape c). Elle peut avantageusement durer par exemple de 1 à 40 heures. On a observé d'excellents résultats en sélectivité en minimisant la concentration en substrat sucré en fin de fermentation, de manière générale à une valeur inférieure à 5 g/l, par exemple de 0,05 à 3 g/l.

Le milieu de fermentation peut comprendre une source d'azote minéral (sous forme d'ions ammonium) et/ou d'azote organique, par exemple sous forme d'aminoacides tels que notamment l'extrait de levure, la peptone de soja, des hydrolysats de caséine, la liqueur de macération du maïs, des hydrolysats de gluten de blé, des extraits de viande. L'addition d'éléments minéraux tels que par exemple le potassium, le sodium, le magnésium et d'oligoéléments comme le fer, le manganèse, le molybdène sous forme de leurs sels (sulfates, phosphates, chlorures) peuvent permettre également d'améliorer encore la croissance.

Les conditions de fermentation sont habituellement les suivantes : température 20-40°C, pH = 5,5 à 8,0. Un bon niveau d'activité a été obtenu à une température comprise entre 28 et 38°C et dans un intervalle de pH de 5,8 à 7,0 et on observe d'excellents niveaux d'activité à une température comprise entre 32 et 35°C et à un pH de 6,0 à 6,8. La fermentation s'effectue dans des conditions d'anaérobiose et dans des conditions de stérilité parfaites. On agite généralement le milieu par des moyens mécaniques connus.

Dans les conditions optimisées citées ci-haut, on a observé des durées de fermentation n'excédant pas par exemple 4 jours avec d'excellents niveaux de production et de sélectivité en acide n-butyrique ou en ses sels avec des apports globaux en substrat sucré généralement compris entre 100 et 350 g/l par litre de milieu réactionnel initial. La durée de fermentation est donc nettement réduite, ce qui implique des capacités de fermenteurs nettement moins importantes pour une production donnée et donc des investissements limités.

Pendant toute la durée de la fermentation, le pH est contrôlé et régulé à une valeur de consigne comprise dans la gamme décrite ci-haut par l'apport d'une solution alcaline $NH_4OH$ par exemple. Par le dégagement gazeux composé de $CO_2$ et $H_2$, on peut suivre l'avancement de la réaction et donc en déduire le moment où on fait démarrer l'alimentation en substrat sucré.

Avantageusement, l'alimentation en substrat sucré est effectuée à partir d'un réservoir de stockage dans lequel la concentration en substrat est élevée, ce qui minimise les volumes réactionnels et facilite par la suite la récupération des produits.

La récupération des produits obtenus selon la présente invention peut être effectuée de la façon suivante :

Une fois la réaction de fermentation terminée (absence de dégagement gazeux), on sépare les microorganismes du moût de fermentation par des techniques connues telles que filtration ou centrifugation. Le moût clarifié obtenu est concentré par exemple par évaporation. Après acidification par addition d'un acide minéral tel que HCl ou $H_2SO_4$ jusqu'à pH compris entre 0,1 et 2, on obtient une solution qui peut ensuite être distillée dans le but de séparer les deux acides organiques présents et d'obtenir l'acide n-butyrique sensiblement pur. Les exemples suivants illustrent le procédé selon l'invention.

Les résultats sont exprimés par les notions suivantes :
- Sélectivité en acide n-butyrique : rapport de la concentration finale en acide n-butyrique sur la concentration finale en acides organiques totaux.
- Productivité en acide n-butyrique : vitesse de production moyenne d'acide n-butyrique par unité de volume de milieu réactionnel final
- Rendement en acide n-butyrique : rapport de la quantité d'acide n-butyrique produit sur la quantité de substrat sucré consommé.

<u>Exemple 1</u> : Fermentation avec alimentation en glucose avec la souche de <u>Clostridium tyrobutyricum</u> I- 776

Cet exemple décrit une fermentation selon l'invention c'est-à-dire que la souche de <u>Clostridium tyrobutyricum</u> I- 776 est ensemencée sur un milieu à faible concentration en glucose. Le glucose est ensuite injecté au cours du temps à vitesse constante.

Le milieu de fermentation est le suivant :

| | |
|---|---|
| - glucose | 5 g/l |
| - extrait de levure | 8,7 g/l |
| - $(NH_4)_2SO_4$ | 1,7 g/l |
| - $KH_2PO_4$ | 2,6 g/l |
| - $MgSO_4$, $7H_2O$ | 1 g/l |
| - $FeSO_4$, $7H_2O$ | 52 mg/l |

2,3 l de ce milieu sont placés dans un fermenteur de laboratoire de 6 l et l'ensemble est stérilisé pendant 40 minutes à 110°C dans un autoclave, puis après refroidissement à 35°C, le pH est ajusté à 5,8 par addition d'ammoniaque à 10 % (poids/volume). Ce milieu est alors ensemencé anaérobiquement avec une préculture de la souche I- 776. La chaîne d'ensemencement est la suivante : un tube de 10 ml du milieu constitué par :

| | |
|---|---|
| - glucose | 30 g/l |
| - extrait de levure | 5 g/l |
| - $(NH_4)_2SO_4$ | 3 g/l |
| - $KH_2PO_4$ | 1,5 g/l |
| - $MgSO_4$, $7H_2O$ | 0,6 g/l |
| - $FeSO_4$, $7H_2O$ | 30 mg/l |
| - $CaCO_3$ | 5 g/l |

est ensemencé à partir d'un tube de conservation de la souche I- 776. Ce tube est cultivé sous anaérobiose à 34°C pendant 24 heures. Il permet alors d'ensemencer une fiole d'erlenmeyer contenant 200 ml du même milieu que ci-dessus décrit mais avec $CaCO_3$ à 10 g/l. Cette fiole est incubée sous anaérobiose à 34°C pendant 15 heures. On en verse ensuite le contenu dans les 2, 3 litres du milieu de fermentation ci-dessus.

Cette fermentation butyrique produit un dégagement gazeux important composé de $CO_2$ et $H_2$. Ce dégagement est mesuré en reliant une sortie du fermenteur à un compteur à gaz volumétrique. Le pH est mesuré à l'aide d'un pH mètre relié à une électrode trempant dans le milieu de fermentation.

Le pH de la fermentation est régulé, par apport d'ammoniaque 10 %, à pH 5,8. L'ammoniaque est injecté par l'intermédiaire d'une électrovanne reliée au pH mètre et constitue à la fois le moyen de régulation et l'apport d'azote nécessaire à la croissance de la souche I- 776 .

Le dégagement gazeux, l'injection d'ammoniaque et la consommation de glucose débutent immédiatement après ensemencement. Il n'y a pas de temps de latence.

Des dosages de glucose sont effectués sur des prélèvements réalisés dans le milieu de fermentation. Quand la valeur de glucose atteint zéro après 6 heures de réaction, on débute l'injection en continu à l'aide d'une pompe péristaltique à un débit de 66 ml/h d'une solution à 360 g/l de glucose, soit un débit d'alimentation horaire de 23,8 g de glucose par litre ou encore de 9,5 g de glucose par litre de milieu réactionnel initial.

Ce débit permet de se trouver dans des conditions non limitantes en glucose dans le fermenteur. Ce débit est fixe pendant toute la durée de l'injection. Dès le branchement de l'alimentation, on constate une accélération de l'activité fermentaire qui se traduit par une vitesse de dégagement gazeux atteignant 9 l/h après 11 heures de réaction, cette vitesse restant sensiblement constante jusqu'à la 18ème heure de réaction puis décroissant progressivement. Le dégagement gazeux cesse après 34 heures. Parallèlement, on constate une augmentation du glucose résiduel dans le milieu dont la concentration finale atteint 53 g/l. Pendant toute la durée de l'étape c qui est de 22 heures, on a injecté 1,4 l de la solution de glucose à 360 g/l, soit 504 g de glucose au total. La production de gaz s'élève à 155 litres. La concentration finale en acide butyrique normal est de 32 g/l de milieu réactionnel final, celle en acide acétique est de 4,5 g/l.

Cet exemple montre que, par la culture de la souche I- 776, sous condition d'alimentation en glucose,

EP 0 350 355 B1

- le temps de latence est inexistant
- la concentration en acide n-butyrique produit est de 32 g/l de milieu réactionnel final. Sachant que celui-ci est de 4,1 l, la production totale d'acide n-butyrique est de 131 g
- l'acide acétique produit représente seulement 12,3 % des acides totaux et l'acide n-butyrique 87,7 %
- le rendement en acide n-butyrique est de 41,5 % par rapport au glucose consommé
- la productivité en acide n-butyrique est de 1,0 g/l/h.

Lorsque le dégagement gazeux cesse, le moût est centrifugé à 13000 t/min soit une accélération de (27000 x g) pendant 20 minutes. Le jus clarifié est concentré par évaporation de telle façon que la concentration en acides totaux qui était de 36,5 g/l dans le moût atteigne 300 g/l. On acidifie le concentrat à pH 1,0 par addition d'acide sulfurique concentré (36 N).

On récupère la phase supérieure dans laquelle on trouve principalement l'acide n-butyrique présent initialement dans le moût de fermentation. On procède à une distillation pour obtenir l'acide n-butyrique pur à 98 %.

Exemple 2 :

On répète l'exemple 1 mais le glucose est dans cet exemple amené par une solution d'hydrolysat enzymatique de farine de blé.

L'hydrolysat de farine est préparé selon le protocole suivant : un lait de farine à 38,3 % de matière sèche est préparé dans l'eau. Après avoir amené le pH à 6,2 avec $NH_4OH$ 2N, il est soumis à une liquéfaction par réaction d'une enzyme $\alpha$-amylase Termamyl 60 (NOVO) à 0,5 g/kg de farine brute pendant 2 heures à 90°C. L'étape suivante est la saccharification réalisée à pH 4,6 (pH ramené avec $H_3PO_4$) par action d'une enzyme amyloglucosidase AMG 150 L (NOVO) à 1,6 ml/kg de farine brute pendant 66 h à 60°C.

L'hydrolysat de farine est alors centrifugé afin d'éliminer le gluten. On obtient une teneur en glucose d'environ 370 à 380 g/l. C'est cette solution qui sert à alimenter le fermenteur durant l'étape c).

Le milieu de fermentation est identique à celui décrit dans l'exemple 1 sauf que la concentration en glucose initiale provenant de l'hydrolysat de farine est de 2 g/l de milieu réactionnel initial.

Lorsque la concentration en glucose dans le milieu réactionnel atteint zéro, on démarre l'alimentation en hydrolysat de farine à un débit horaire correspondant à 8 g de glucose/l de milieu réactionnel initial. Pendant toute la durée de l'alimentation (étape c) qui est de 37 heures, on a injecté 1,7 l d'hydrolysat de farine à 370 g/l de glucose.

Les résultats et les conditions de fermentation sont consignés dans le tableau n° 1.

Exemple 3 :

On répète l'exemple 1 mais le glucose est amené par l'hydrolysat de farine et sa concentration initiale dans le fermenteur est de 50 g/l. Les conditions opératoires et les résultats sont consignés dans le tableau 1.

Exemple 4 :

On répète l'exemple 1 mais le glucose est amené par l'hydrolysat de farine à la concentration initiale de 6,5 g/l, et l'extrait de levure est remplacé par de l'extrait sec de liqueur de macération de maïs à même concentration, toutes autres conditions étant identiques à celles de l'exemple 1.

Quand la valeur de glucose atteint 3,5 g/l de milieu réactionnel, on branche l'alimentation en substrat de manière à ce qu'elle soit asservie à la vitesse de réaction et en particulier à la vitesse de production du mélange gazeux $H_2$ + $CO_2$. Ceci est réalisé par l'asservissement de la pompe d'injection du substrat à un compteur volumétrique mesurant le flux gazeux, via un microordinateur de type Apple qui calcule la vitesse de dégagement gazeux moyen à chaque nouveau litre de mélange gazeux dégagé. Le microordinateur commande directement la vitesse d'injection à raison de 2,7 g de glucose par litre de gaz dégagé.

Les conditions opératoires et les résultats sont consignés dans le tableau 1.

Exemple 5 : Comparatif

Selon l'art antérieur, on a procédé à une fermentation conventionnelle (apport de substrat réalisé en totalité au temps zéro) avec la souche de Clostridium tyrobutyricum I- 776 . La quantité de glucose globale provenant d'hydrolysats de farine est de 130 g/l de volume réactionnel initial soit une quantité sensiblement

5

égale à celles injectées lors des étapes c présentées dans les exemples 1 à 4 selon l'invention. Le milieu réactionnel est identique à celui de l'exemple 1.

Les résultats sont présentés dans le tableau 1.

Exemple 6 :

On répète l'exemple 1 mais on remplace la souche de Clostridium tyrobutyricum I- 776 par la souche de Clostridium tyrobutyricum I - 775.

Les conditions opératoires et les résultats sont présentées dans le tableau 1.

Exemple 7 :

On répète l'exemple 6 mais la concentration initiale en glucose est de 80 g/l de milieu réactionnel initial.

Les conditions opératoires et les résultats sont présentés dans le tableau 1.

Exemple 8 :

On répète l'exemple 1 mais on remplace la souche de Clostridium tyrobutyricum I- 776 par la souche de Clostridium butyricum IFP 925.

Les conditions opératoires et les résultats sont présentés dans le tableau 1.

Exemple 9 : Comparatif

Selon l'art antérieur, on a procédé à une fermentation en batch comme dans l'exemple 5 mais avec la souche de Clostridium butyricum IFP 925. La quantité globale de glucose fournie en totalité au temps zéro est de 80 g/l de milieu réactionnel initial.

Les résultats sont présentés dans le tableau 1.

TABLEAU 1

CONDITIONS OPERATOIRES ET RESULTATS

| N° de l'exemple | Souche | Substrat sucré | Conditions opératoires | | | | | Résultats | | | |
|---|---|---|---|---|---|---|---|---|---|---|---|
| | | | Concentration initiale en glucose (g/l) | Concentration en glucose au démarrage de l'étape c) (g/l) | Débit d'alimentation en glucose (g/l/h) | Durée de l'étape c) (h) | Concentration résiduelle minimum en glucose durant l'étape c) (g/l) | Concentration finale en acide n-butyrique (g/l) | Sélectivité en acide n-butyrique (%) | Productivité en acide n-butyrique g/l/h | rendement en acide n-butyrique % |
| 1 | I-776 | Glucose | 5 | 0 | 9,5 | 22 | 53 | 32 | 87,7 | 1,00 | 41,5 |
| 2 | I-776 | Glucose * | 2 | 0 | 8 | 37 | 39 | 64,5 | 100,0 | 0,97 | 45,0 |
| 3 | I-776 | Glucose * | 50 | 0 | 10,8 | 26 | - | 55,8 | 96,0 | 1,15 | 41,9 |
| 4 | I-776 | Glucose * | 8,5 | 3,5 | 0,55 - 11,35 | 52 | 25 | 55 | 94,9 | 0,57 | 40,6 |
| 5 | I-776 | Glucose * | 130 | - | - | - | - | 40,6 | 75,0 | 0,33 | 39,0 |
| 6 | I-775 | Glucose | 5 | 0 | 13,4 | 18 | 60 | 47,8 | 95,4 | 0,68 | 36,7 |
| 7 | I-775 | Glucose | 80 | 20 | 13,2 | 15 | 67 | 42,5 | 90,4 | 0,82 | 35,7 |
| 8 | IFP 925 | Glucose | 5 | 0 | 5,6 | 24 | 18,7 | 26,5 | 82,5 | 0,95 | 33,0 |
| 9 | IFP 925 | Glucose | 80 | - | - | - | - | 23,7 | 66,6 | 1,07 | 35,5 |

* Glucose apporté par l'hydrolysat enzymatique de farine de blé

**Revendications**

1.  Procédé de production, dans une zone réactionnelle, d'un mélange d'acides organiques ou des sels correspondants contenant de 80 à 100% d'acide n-butyrique ou de son sel correspondant, par fermentation, dans des conditions de fermentation d'un milieu réactionnel initial contenant un substrat sucré, en présence d'au moins une souche appartenant à l'espèce Clostridium, caractérisé en ce que :
    a) on alimente ladite souche en ledit substrat à une concentration initiale comprise entre 1 et 80 g de substrat par litre de milieu réactionnel initial,
    b) on laisse diminuer la concentration en substrat d'au moins 20 % de sa valeur initiale et jusqu'à une valeur telle qu'elle soit comprise entre 0 et 30 g de substrat par litre de milieu réactionnel initial,
    c) on alimente ladite souche en ledit substrat à un débit compris entre 0,01 et 25 g par litre de milieu réactionnel initial et par heure et pendant une durée d'alimentation tels que la concentration résiduelle en ledit substrat pendant la durée d'alimentation est au plus égale à 80 g/l de milieu réactionnel initial, et
    d) on récupère ledit mélange d'acides ou de sels ainsi produit.

2.  Procédé selon la revendication 1, dans lequel ladite souche est choisie dans le groupe formé par Clostridium tyrobutyricum, Clostridium butyricum, Clostridium pasteurianum, Clostridium acetobutylicum et Clostridium beijerinckii.

3.  Procédé selon les revendications 1 ou 2, dans lequel la souche est Clostridium tyrobutyricum I - 776 ou Clostridium tyrobutyricum I-775.

4.  Procédé selon l'une des revendications 1 à 3, dans lequel le substrat sucré comporte des hydrates de carbone sous forme monosaccharide provenant de l'hydrolyse acide ou enzymatique des disaccharides et/ou polysaccharides contenus dans les matières premières choisies dans le groupe formé par les jus sucrés obtenus par pressage ou diffusion des betteraves, de la canne à sucre et/ou du topinambour, les sirops, les égouts et les mélasses de sucrerie, les farines de céréales, les farines de maïs et la fécule de pomme de terre.

5.  Procédé selon l'une des revendications 1 à 4 dans lequel la concentration initiale en ledit substrat est comprise entre 1 et 30 g/l de milieu réactionnel initial et de manière préférée comprise entre 2 et 10 g/l.

6.  Procédé selon l'une des revendications 1 à 5 dans lequel la concentration en ledit substrat au terme de l'étape b) est comprise entre 0 et 15 g/l de milieu réactionnel et de préférence comprise entre 0 et 6 g/l.

7.  Procédé selon l'une des revendications 1 à 6 dans lequel le débit d'alimentation en substrat de l'étape c) est compris entre 2 et 20 g par litre de milieu réactionnel initial et par heure et de préférence entre 5 et 15 g/l/h.

8.  Procédé selon l'une des revendications 1 à 7 dans lequel ladite concentration résiduelle en substrat pendant l'étape (c) est comprise entre 0 et 40 g par litre de milieu réactionnel initial.

9.  Procédé selon l'une des revendications 1 à 8 dans lequel la récupération du mélange d'acides ou de sels comprend une étape de séparation de ladite souche et d'une solution contenant ledit mélange, une étape de concentration de ladite solution, une étape d'acidification de ladite solution à pH compris entre 0,1 et 2 délivrant une phase supérieure contenant ledit mélange d'acides que l'on sépare et une étape de distillation du mélange ainsi séparé.

10. Procédé selon l'une des revendications 1 à 9 dans lequel ledit mélange renferme de 80 à 100 % d'acide n-butyrique ou de son sel correspondant et de 20 à 0 % d'acide acétique ou de son sel et avantageusement de 90 à 100 % d'acide n-butyrique ou de son sel et de 10 à 0 % d'acide acétique et/ou de son sel.

## Claims

1. Process for the production, in a reaction zone, of a mixture of organic acids or the corresponding salts containing 80 to 100 % n-butyric acid or its corresponding salt, by fermentation, under fermentation conditions of an initial reaction medium containing a sugar substrate, in the presence of at least strain belonging to the Clostridium species, characterized in that:
   a) said strain is supplied to said substrate at an initial concentration between 1 and 80g of substrate per litre of initial reaction medium,
   b) the substrate concentration is allowed to decrease by at least 20% of its initial value and up to a value such that it is between 0 and 30g of substrate per litre of initial reaction medium,
   c) said strain is supplied to said substrate at a flow rate between 0.01 and 25g per litre of initial reaction medium and per hour and for a supply time such that the residual concentration of said substrate during the supply time is at the most equal to 80g/l of initial reaction medium and
   d) recovery takes place of said mixture of acids or salts produced in this way.

2. Process according to claim 1, wherein said strain is chosen from within the group formed by Clostridium tyrobutyricum, Clostridium butyricum, Clostridium pasteurianum, Clostridium acetobutylicum and Clostridium beijerinckii.

3. Process according to claims 1 or 2, wherein the strain is Clostridium tyrobutyricum I-776 or Clostridium tyrobutyricum I-775.

4. Process according to one of the claims 1 to 3, wherein the sugar substrate incorporates carbohydrates in monosaccharide form obtained from the acid or enzymatic hydrolysis of disaccharides and/or polysaccharides contained in the starting substances chosen from within the group formed by sugar-containing juices obtained by the pressing or diffusion of sugarbeet, sugar cane and/or Jerusalem artichokes, syrups and molasses of sugar production, cereal flours, maize flour and potato starch.

5. Process according to one of the claims 1 to 4, wherein the initial concentration of said substrate is between 1 and 30g/l of initial reaction medium and in preferred manner between 2 and 10g/l.

6. Process according to one of the claims 1 to 5, wherein the concentration of said substrate relative to stage b) is between 0 and 15g/l and preferably between 0 and 6g/l of reaction medium.

7. Process according to one of the claims 1 to 6, wherein the substrate supply rate of stage c) is between 2 and 20 and preferably between 5 and 15g/litre of initial reaction medium and per hour.

8. Process according to one of the claims 1 to 7, wherein said residual substrate concentration in stage c) is between 0 and 40g per litre of initial reaction medium.

9. Process according to one of the claims 1 to 8, wherein the recovery of the mixture of acids or salts comprises a stage of separating said strain and a solution containing said mixture, a stage of concentrating said solution, a stage of acidifying said solution to a pH between 0.1 and 2 providing an upper phase containing said mixture of acids, which is separated and a stage of distilling the thus separated mixture.

10. Process according to one of the claims 1 to 9, wherein said mixture contains 80 to 100% n-butyric acid or its corresponding salt and 20 to 0% of acetic acid or its salt and advantageously 90 to 100% n-butyric acid or its salt and 10 to 0% acetic acid and/or its salt.

## Patentansprüche

1. Herstellungsverfahren in einer Reaktionszone eines Gemisches organischer Säuren oder der korrespondierenden Salze, das 80 bis 100 % n-Butansäure oder ihr korrespondierendes Salz enthält, durch Fermentation unter Fermentationsbedingungen in einem Ausgangsreaktionsmedium, das ein Zuckersubstrat umfaßt, in Gegenwart von zumindest einem Stamm, der von der Spezies Clostridium abstammt, dadurch gekennzeichnet daß,

a) der besagte Stamm in dem besagten Substrat mit einer Ausgangskonzentration, die zwischen einschließlich 1 und 80 g Substrat pro Liter des Ausgangsreaktionsmediums liegt, ernährt wird,

b) man die Substratkonzentration um mindestens 20 % ihres Ausgangswertes und bis auf einen Wert, der zwischen einschließlich 0 und 30 Gramm des Substrates pro Liter des Ausgangsreaktionsmediums liegt, absinken läßt,

c) der besagte Stamm in dem besagten Substrat mit einem Durchsatz zwischen einschließlich 0.01 und 25 g pro Liter des Ausgangsreaktionsmediums und pro Stunde und während einer Ernährungsdauer, die so bemessen ist, daß die restliche Konzentration an dem besagten Substrat während der Ernährungsdauer höchstens gleich 80 g/l des Ausgangsreaktionsmediums beträgt, ernährt wird, und

d) das auf diese Art produzierte besagte Gemisch von Säuren oder ihrer Salze gewonnen wird.

2. Verfahren nach Anspruch 1, bei dem der besagte Stamm aus der von Clostridium tyrobutyricum, Clostridium butyricum, Clostridium pasteurianum, Clostridium acetobutylicum und Clostridium beijerinckii gebildeten Gruppe gewählt wird.

3. Verfahren nach den Ansprüchen 1 oder 2, bei denen der Stamm Clostridium tyrobutyricum I-776 oder Clostridium tyrobutyricum I-775 ist.

4. Verfahren nach einem der Ansprüche 1 bis 3, bei denen das Zuckersubstrat die Kohlehydrate in Form von Monosacchariden umfaßt, die aus der sauren oder enzymatischen Hydrolyse von Disacchariden und/oder Polysacchariden, die in den Ausgangsmaterialien enthalten sind, die aus der von den zuckerhaltigen Säften, die durch Raspeln oder Auspressen von Rüben, von Zuckerrohr und/oder Topinambur erhalten werden, den Sirupen, den Abwässern und den Melassen aus der Zuckerherstellung, Getreidemehlen, Maismehl und Kartoffelstärke gebildeten Gruppe gewählt werden.

5. Verfahren nach einem der Ansprüche 1 bis 4, bei dem die Ausgangskonzentration an besagtem Substrat zwischen einschließlich 1 und 30 g/l des Ausgangsreaktionsmediums und vorteilhafterweise zwischen einschließlich 2 und 10 g/l liegt.

6. Verfahren nach einem der Ansprüche 1 bis 5, bei dem die Konzentration an besagtem Substrat am Ende des Schrittes b) zwischen einschließlich 0 und 15 g/l des Ausgangsreaktionsvolumens und vorzugsweise zwischen einschließlich 0 und 6 g/l liegt.

7. Verfahren nach einem der Ansprüche 1 bis 6, bei dem der Versorgungsdurchsatz mit Substrat beim Schritt c) zwischen einschließlich 2 und 20 g pro Liter des Ausgangsreaktionsmediums und pro Stunde und vorzugsweise zwischen 5 und 15 g/l/h liegt.

8. Verfahren nach einem der Ansprüche 1 bis 7, bei dem die besagte Restkonzentration an Substrat während des Schrittes c) zwischen einschließlich 0 und 40 g pro Liter des Ausgangsreaktionsmediums liegt.

9. Verfahren nach einem der Ansprüche 1 bis 8, bei dem das Gewinnen des Gemisches von Säuren oder Salzen einen Schritt zur Trennung des besagten Stammes und einer das besagte Gemisch enthaltenen Lösung, einen Schritt zur Aufkonzuentrierung der besagten Lösung, einen Ansäuerungsschritt der besagten Lösung auf einen pH-Wert zwischen einschließlich 0,1 und 2, der eine obere, das besagte Säuregemisch enthaltende Phase liefert, die abgetrennt wird, und einen Schritt zur Destillation des auf diese Weise gewonnen Gemisches umfaßt.

10. Verfahren nach einem der Ansprüche 1 bis 9, bei dem das besagt Gemisch 80 bis 100 % n-Butansäure oder ihr korrespondierendes Salz und 20 bis 0 % Essigsäure oder ihr Salz und vorteilhafterweise 90 bis 100 % n-Butansäure oder ihr Salz und 10 bis 0 % Essigsäure und/oder ihr Salz umfaßt.